# EUROPEAN PATENT SPECIFICATION

(11) **EP 3 648 833 B1**
(45) Date of publication and mention of the grant of the patent: **04.08.2021**
(21) Application number: 17916203.7
(22) Date of filing: 29.06.2017
(51) Int. Cl.: A61N 1/05, A61B 5/11, A61H 19/00, A61H 21/00, A61N 1/00, A61N 1/08, A61N 1/36

(54) **DEVICE USING ELECTROTHERAPY FOR THE RELIEF OF HEMORRHOID INFLAMMATION**
VORRICHTUNG MIT ELEKTROTHERAPIE ZUR LINDERUNG VON HÄMORRHOIDENENTZÜNDUNG
DISPOSITIF UTILISANT L'ÉLECTROTHÉRAPIE POUR LE SOULAGEMENT D'UNE INFLAMMATION HÉMORROÏDAIRE

(43) Date of publication of application: 13.05.2020
(73) Proprietor: Hemoref, Inc., Tampa FL 33647 (US)
(72) Inventor: DOMINGUEZ, Yamil, Tampa, FL 33647 (US)
(74) Representative: Urizar Anasagasti, Jesus Maria
(86) International application number: PCT/US2017/039942
(87) International publication number: WO 2019/005060

(56) References cited:
- US-A1- 2006 074 356
- US-A1- 2009 222 058
- US-A1- 2014 194 946
- US-A1- 2014 222 102
- US-A1- 2015 134 026
- US-A1- 2015 328 082
- US-A1- 2016 045 745
- US-A1- 2016 263 387

## Description

### II. BACKGROUND OF THE INVENTION

### 1. Field of the Invention.

The present invention relates to a device to treat hemorrhoid inflammation and, more particularly, to such a device that uses electrotherapy to relieve hemorrhoid inflammation.

### 2. Description of the Related Art.

Several designs for a hemorrhoid relief device have been designed in the past. None of them, however, include a device that can be connected wirelessly or wired to a control unit to provide electrotherapy stimulation to a user's anus, thereby relieving inflammation and accompanying discomfort.

Applicant believes that a related reference corresponds to U.S. patent application No. US20120029602 filed by Vascular Technologies, Inc. for a method, apparatus, and system for treatment of hemorrhoidal disease using negative galvanism. However, it differs from the present invention because the *Vascular* reference teaches of a device that must be held against the anus by a user. The user does not have his hands free but instead must intrusively maintain the device in the desired position notwithstanding the fatigue associated with doing so. The present invention addresses this unsolved problem by creating a capsule that is inserted into a user's anus a predetermined depth. The capsule is connected to a control unit where a user can modulate the amount of stimulation without affecting the placement of the device. With the prior art the device would have to be removed from the inflammation site to be modulated. Futhermore, US 2006074356 A1 discloses a probe module for treating prostate diseases and portable prostate medical treatment apparatus.

Other documents describing the closest subject matter provide for a number of more or less complicated features that fail to solve the problem in an efficient and economical way. None of these patents suggest the novel features of the present invention.

### III. SUMMARY OF THE INVENTION

It is one of the main objects of the present invention to provide a device that relieves hemorrhoid inflammation using electrotherapy.

It is another object of this invention to provide such a device that is inserted into a user's anus allowing hands free operation.

It is still another object of the present invention to provide such a device having a control unit that is connected wired or wirelessly to a capsule inserted into a user's anus that can be modulated to a desired intensity determined by the user.

It is yet another object of this invention to provide such a device that is inexpensive to implement and maintain while retaining its effectiveness.

Further objects of the invention will be brought out in the following part of the specification, wherein detailed description is for the purpose of fully disclosing the invention without placing limitations thereon.

### IV. BRIEF DESCRIPTION OF THE DRAWINGS

With the above and other related objects in view, the invention consists in the details of construction and combination of parts as will be more fully understood from the following description, when read in conjunction with the accompanying drawings in which:
**Figure 1** represents a front elevational view of the device having the control unit mounted to the lower back of the user and connected to a capsule inserted into a user's anus through a wire.

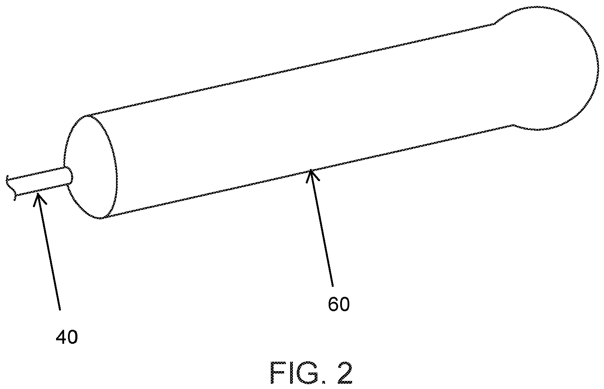

**Figure 2** shows an isometric view of the capsule without the wire or control unit shown.
**Figure 3** illustrates a front elevational view of the control unit showing the power switch, power indicator, touchscreen display, and buttons used to modulate the intensity and/or the duration and rhythm of electrotherapy.
**Figure 4** is a representation of an alternate embodiment of the present invention wherein the control module includes a modulating dial instead of a touchscreen to adjust the intensity of the electrotherapy.

### V. DETAILED DESCRIPTION OF THE EMBODIMENTS OF THE INVENTION

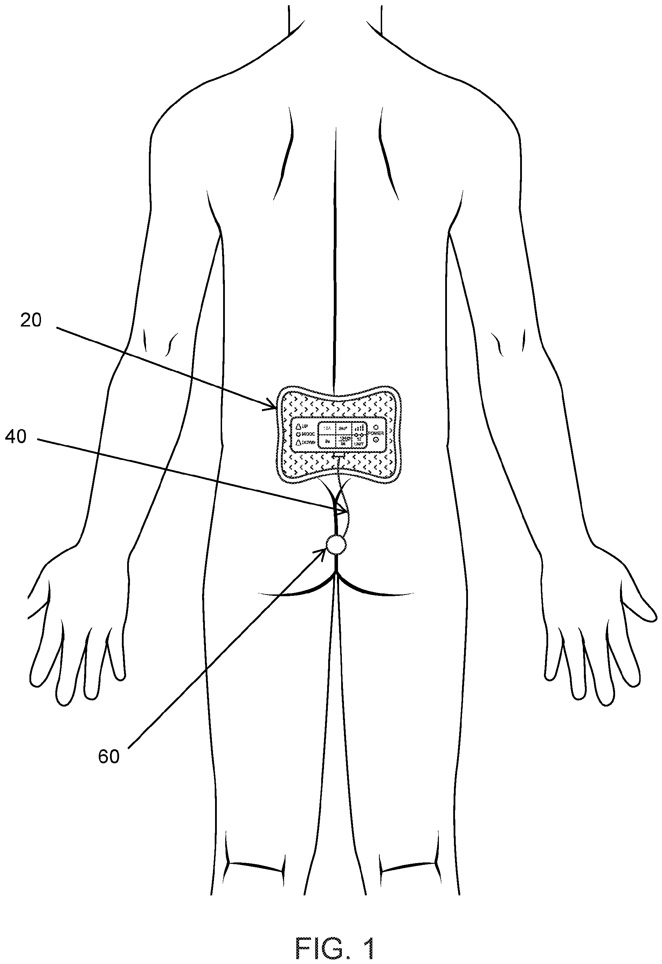

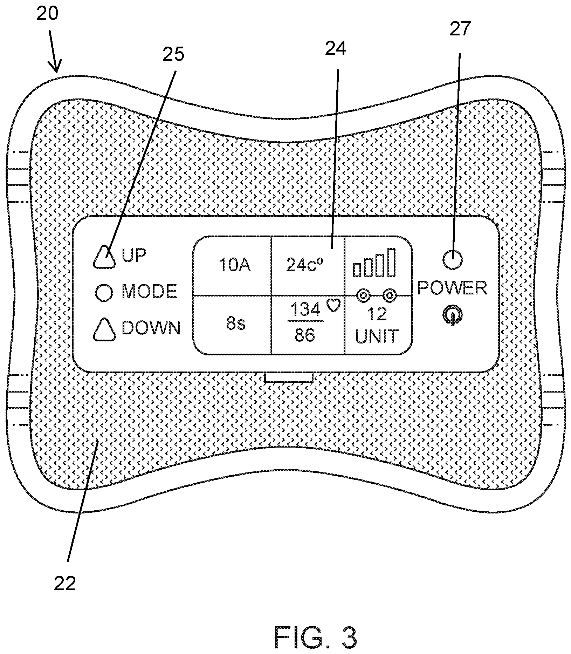

Referring now to the drawings, where the present invention is generally referred to with numeral **10**, it can be observed that it basically includes control unit assembly **20**, connection means **40**, and capsule assembly **60**. As shown in figures 1, 3 and 4, control unit assembly **20** includes control unit member **22** being mounted to a predetermined location on a user's body such as the lower back. Control unit member **22** can be mounted to the user's body using a strap, adhesive gel, acrylate, including methacrylates and epoxy diacrylates (which are also known as vinyl resins), or similar means. Control unit member **22** further includes a plurality of sensors and reading displays **24**. Intensity control buttons **25** are used to increase or decrease the intensity of the electrotherapy being transmitted to capsule assembly **60**. Power button **27** is also used to activate the device.

Mode control button **26** is used to alternate the rhythm and pattern that the electrotherapy is transmitted to capsule **60**. Reading displays **24** can provide various information such as heart rate, blood pressure, intensity level, body temperature, duration of the therapy, etc. These readings can be done with corresponding sensors located on the inside of control unit member **22**.

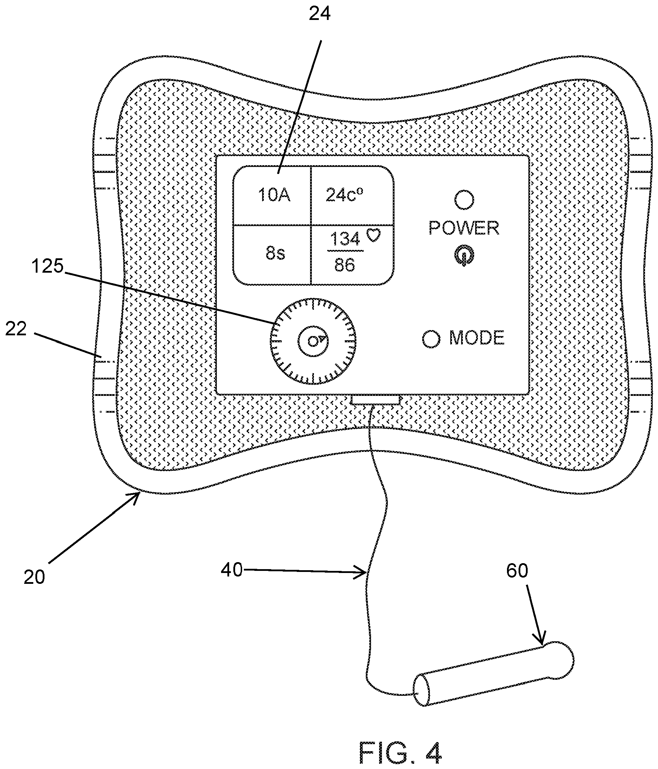

As shown in figure 4, control unit assembly **20** is connected to capsule assembly **60** using connection means **40**. Capsule assembly **60** has a shape that is configured to cooperate with the anus of a user so that it can be inserted comfortably and snuggly. Capsule assembly **60** is of a predetermined length and width to interact with the areas that require relief due to hemorrhoidal inflammation. Electrical energy is transmitted to capsule **60** using connection means **40** to provide electrotherapy to a user.

Control unit member **22** includes an internal rechargeable battery that acts as the source of the electrical energy. In one embodiment, the battery can be recharged using a USB port on control unit member **22**. The battery can also be charged using a charging dock. In one embodiment, shown in figure 4, intensity buttons **25** can be in analog form using a dial **125**. Connection means **40** is of a length that cooperates with the distance between control unit member **22** and capsule assembly **60**.

In one embodiment, a user can control intensity buttons **25** and power button **27** using a mobile application on their mobile device. Using the mobile application a user can turn on and off the device or increase or decrease the intensity of electrical energy. Also, the readings on display readings **24** can be displayed on the mobile application. In another embodiment, connection means **40** is wireless and capsule **60** can be fitted with its own battery so that electrotherapy is originated in capsule **60** and delivered straight to the user therefrom. In this embodiment, capsule **60** can include a Bluetooth receiver that receives instructions from a Bluetooth transmitter on a remote control or through a mobile application operated by a user.

The foregoing description conveys the best understanding of the objectives and advantages of the present invention. Different embodiments may be made of the inventive concept of this invention. The invention is defined by independent claim 1.

### VI. INDUSTRIAL APPLICABILITY

The present invention's industrial applicability is that it functions to relieve a user's hemorrhoids by stimulating the area that has discomfort using a patch-like control unit that can be used to modulate the intensity of the therapy provided by the device through the capsule inserted into a user's anus having the therapy providing electrodes therein.

## Claims

1. A device to relieve hemorrhoids comprising: a capsule that is adapted to be inserted into a user's anus, a patch-like control unit member with electrotherapy intensity modulation, said capsule includes electrodes to provide electrotherapy configured to relieve hemorrhoids, said capsule having a shape that allows it to remain in place on its own within the anus said capsule wirelessly connected to said control unit member, said control unit member configured to be attached to a user's back, said control unit controlling an intensity of the electrotherapy provided by the capsule inside said user's anus, a downloadable software program on a mobile device used to modulate said electrotherapy, wherein the mobile device is a separate component from the control unit member.

2. The device subject of claim 1 wherein a remote control is used to modulate the electrotherapy delivered by said capsule, said remote also powers on and off the electrotherapy.

3. The device subject of claim 1 wherein intensity buttons on said control unit or software increase and decrease the intensity of electrotherapy delivered to a user.

4. The device subject of claim 1 wherein said capsule or said control unit include a battery.

5. The device subject of claim 1 wherein said intensity buttons are digital or analog.

6. The device subject of claim 1 wherein said control unit includes sensors that read a user's blood pressure, heart rate, therapy duration, or body temperature.

7. The device of claim 1 wherein said capsule is connected to said control unit using a wired connection.

8. The device of claim 1 wherein said control unit is mounted to a user's back using an adhesive.

9. The device of claim 1 wherein said control unit is mounted to a user's back using a strap.

## Patentansprüche

1. Verfahren zum Lindern von Hämorrhoiden, umfassend: eine Kapsel, die ausgelegt ist, um in einen Anus eines Benutzers eingeführt zu werden, ein pflasterartiges Steuereinheitselement mit Elektrotherapie-Intensitätsmodulation, wobei die Kapsel Elektroden einschließt, um eine Elektrotherapie bereitzustellen, die konfiguriert ist, um Hämorrhoiden zu lindern, wobei die Kapsel eine Form aufweist, die es ihr ermöglicht, von selbst im Anus zu verbleiben, wobei die Kapsel drahtlos mit dem Steuereinheitselement verbunden ist, wobei das Steuereinheitselement zum Anbringen an einem Rücken eines Benutzers konfiguriert ist, wobei die Steuereinheit eine Intensität der Elektrotherapie steuert, die von der Kapsel innerhalb des Anus des Benutzers bereitgestellt wird, wobei ein herunterladbares Softwareprogramm auf einer mobilen Vorrichtung verwendet wird, um die Elektrotherapie zu modulieren, wobei die mobile Vorrichtung eine separate Komponente von dem Steuereinheitselement ist.

2. Vorrichtung, Gegenstand von Anspruch 1, wobei eine Fernbedienungssteuerung verwendet wird, um die Elektrotherapie zu modulieren, die von der Kapsel abgegeben wird, wobei die Fernsteuerung die Elektrotherapie auch ein- und ausschaltet.

3. Vorrichtung, Gegenstand von Anspruch 1, wobei die Intensitätstaster auf der Steuereinheit oder Software die an einen Benutzer abgegebene Intensität der Elektrotherapie erhöhen oder verringern.

4. Vorrichtung, Gegenstand von Anspruch 1, wobei die Kaspel der Steuereinheit eine Batterie einschließt.

5. Vorrichtung, Gegenstand von Anspruch 1, wobei die Intensitätstaster digital oder analog sind.

6. Vorrichtung, Gegenstand von Anspruch 1, wobei die Steuereinheit Sensoren einschließt, die einen Blutdruck, einen Herzschlag, eine Therapiedauer oder Körpertemperatur des Benutzers lesen.

7. Vorrichtung nach Anspruch 1, wobei die Kaspel mit der Steuereinheit verbunden ist, die eine verdrahtete Verbindung verwendet.

8. Vorrichtung nach Anspruch 1, wobei die Steuereinheit an einem Rücken des Benutzers unter Verwendung eines Haftmittels angebracht ist.

9. Vorrichtung nach Anspruch 1, wobei die Steuereinheit an einem Rücken des Benutzers unter Verwendung eines Riemens angebracht ist.

## Revendications

1. Dispositif pour soulager les hémorroïdes comprenant : une capsule qui est adaptée pour être insérée dans l'anus d'un utilisateur, un élément d'unité de commande en forme de patch avec modulation d'intensité d'électrothérapie, ladite capsule inclut des électrodes pour fournir une électrothérapie configurée pour soulager les hémorroïdes, ladite capsule ayant une forme qui lui permet de rester en place par elle-même à l'intérieur de l'anus ladite capsule étant connectée sans fil audit élément d'unité de commande, ledit élément d'unité de commande étant configuré pour être fixé au dos d'un utilisateur, ladite unité de commande commandant une intensité de l'électrothérapie fournie par la capsule à l'intérieur dudit anus de l'utilisateur, un logiciel téléchargeable sur un appareil mobile utilisé pour moduler ladite électrothérapie, dans lequel le dispositif mobile est un composant séparé de l'élément d'unité de commande.

2. Dispositif objet de la revendication 1 dans lequel une télécommande est utilisée pour moduler l'électrothérapie délivrée par ladite capsule, ladite télécommande allume et éteint également l'électrothérapie.

3. Dispositif objet de la revendication 1 dans lequel les boutons d'intensité sur ladite unité de commande ou logiciel augmentent et diminuent l'intensité de l'électrothérapie délivrée à un utilisateur.

4. Dispositif objet de la revendication 1 dans lequel ladite capsule ou ladite unité de commande inclut une batterie.

5. Dispositif objet de la revendication 1 dans lequel lesdits boutons d'intensité sont numériques ou analogiques.

6. Dispositif objet de la revendication 1 dans lequel ladite unité de commande inclut des capteurs qui lisent la tension artérielle, le rythme cardiaque, la durée de thérapie, ou la température corporelle d'un utilisateur.

7. Dispositif objet de la revendication 1 dans lequel ladite capsule est connectée à ladite unité de commande en utilisant une connexion filaire.

8. Dispositif objet de la revendication 1 dans lequel ladite unité de commande est montée sur le dos d'un utilisateur en utilisant un adhésif.

9. Dispositif objet de la revendication 1 dans lequel ladite unité de commande est montée sur le dos d'un utilisateur à l'aide d'une sangle.
